# EUROPEAN PATENT APPLICATION

(11) **EP 1 112 749 A2**
(43) Date of publication of application: **04.07.2001**
(21) Application number: 99937066.1
(22) Date of filing: 12.08.1999
(51) Int. Cl.: A61K 45/00, A61K 31/66, A61K 31/40, A61K 31/445, A61K 31/415, A61K 31/505, A61K 31/53

(54) **PREVENTIVES OR REMEDIES FOR EYE CIRCULATORY FAILURE**

(30) Priority: 20.08.1998 JP 25193198
(71) Applicant: Senju Pharmaceutical Co., Ltd., Osaka-shi, Osaka 541-0046 (JP)
(72) Inventor: INOUE, Jun, Kobe-shi, Hyogo 654-0101 (JP); WAKI, Mitsunori, Kobe-shi, Hyogo 651-2227 (JP)
(74) Representative: von Kreisler, Alek, Dipl.-Chem.
(86) International application number: JP9904395
(87) International publication number: WO0010605

(57) **Abstract**

The conventional pharmaootherapy for diseases caused by a disturbance of ocular circulation, inclusive of glaucoma, comprises oral administration of steroids and other drugs and/or topical administration of β -adrenergic antagonists but these therapeutic regimens are invariably not effective enough but rather entail adverse reactions characteristic of the respective drugs used.

This invention is characterized in that a chymase inhibitor is used for the prevention or treatment of diseases caused by a disturbance of ocular circulation and enables positive prophylaxis or therapy with reduced risks for side effects.

## Description

### TECHNICAL FIELD

The present invention relates to a prophylactic or therapeutic medicament for various diseases caused by a disturbance of ocular circulation such as intraocular blood circulation and aqueous circulation. The present invention further provides novel uses for chymase inhibitors.

### BACKGROUND ART

It is known that angiotensin II induces a marked elevation of blood pressure, constricts blood vessels, and releases aldosterone, a sodium-retaining hormone, from the adrenal gland.

It is also considered likely that angiotensin II is a causative agent or a risk factor in other diseases associated with hypertension [for example, hypercardia (ectasia cordis), myocardial infarction, vascular hypertrophy (migration or proliferation of vascular smooth muscle cells or hyperplasia of extracellular matrix), diabetic or nondiabetic nephropathy (partly caused by glomerular hypertension), and vascular restenosis following angioplasty or thrombolytic therapy in patients with atherosclerosis, etc.]. Since angiotensin II is produced from angiotensin I by angiotensin-converting enzyme (ACE), the pathway with which ACE is known to be associated has been the target of many clinically useful antihypertensive drugs.

However, it has recently been elucidated that, aside from said ACE pathway, the chymase constituting a subfamily of serine protease, inclusive of human heart chymase, mast cell chymase, skin chymase, etc., cleaves angiotensin I to give angiotensin II [J. Hypertension, 2, 277 (1984)].

Human chymase provides its own pathway independently of ACE for the production of angiotensin II and, therefore, is likely to be a cause for certain types of hypertension and congestive heart failure. Moreover, in addition to the production of angiotensin II, the chymase activates collagenase to promote migration of smooth muscle cells and endothelial cells or stimulates cytokines such as IL-1β, thus being suspected to be an etiologic factor in degenerative diseases accompanied by organ and vascular lesions. To treat or prevent such damages to the organs or blood vessels in addition to lowering blood pressure should constitute a useful therapy for cardiovascular diseases such as hypertension, and administering a substance capable of suppressing the catalytic activity of human chymases is considered to be a rewarding approach toward remission or prophylaxis of these diseases.

According to the above reasoning, several chymase-inhibitory substances have been reported in WO 93-25574, USP5306824 and WO96-4248. However, there has not been even a report on the influence of chymase inhibitors on the ocular circulation.

The onset of diseases due to a disturbance of ocular circulation is generally thought to be evoked by various factors or a complicated interplay of such factors but the actualities involved have not been fully elucidated as yet.

The disease caused by a disturbance of ocular circulation includes, among others, chorioretinal diseases (e.g. retinal vascular abnormalities such as retinal vascular occlusion, retinal periphlebitis, Eales' disease, ischemic eye syndrome, retinal arteriolar microaneurysm, etc., retinopathies associated with hypertension, renal disease or hemodyscrasia, diabetic retinopathy, retinal pigment epitheliopathy, retinal dystrophy, macular dystrophy, retinochoroidal atrophy, chorioretinopathy, macular degeneration, macular edema, detachment of retinal pigment epithelium, retinal detachment, degenerative retinoschisis, various tumors such as retinoblastoma, retinal pigment epithelioma, optic disc capillary hemangioma, etc., optic neuropathies such as ischemic optic neuropathy, optic disc swelling such as choked disc, papilledema, etc.) and glaucoma (e.g. open-angle glaucoma, low-tension glaucoma, closed-angle glaucoma, etc.). The current pharmacotherapy for diseases caused by a disturbance of ocular circulation includes oral administration of peripheral circulation-improving agents such as tocopherol nicotinate, which is a vitamin E preparation, pentoxiphylline, etc., various steroids, anti-prostaglandin agents, anti-inflammatory enzymes, etc. but these drugs are either not effective enough or known to have side effects such as hypotension and gastrointestinal disorders. The therapy of glaucoma includes administration of cholinergic agonists represented by pilocarpine, sympathomimetic drugs (adrenergic agonists) such as epinephrine, dipivefrin, etc., β -adrenergic antagonists (β -blockers) such as timolol, pindolol, carteolol, etc. but the characteristic side effects of these respective drugs are of serious concern.

The present invention has for its object to provide a prophylactic or therapeutic medicament for diseases caused by a disturbance of ocular circulation, with which the above disadvantages can be solved. The present invention further relates to a method for prophylaxis or therapy of diseases arising from a disturbance of ocular circulation. As used in this specification, the term "ocular circulation" means the intraocular blood circulation and aqueous circulation.

### DISCLOSURE OF INVENTION

The inventors of the present invention made intensive investigations for developing a prophylactic or therapeutic medicament for diseases caused by a disturbance of ocular circulation which is not only capable of producing an effective remission in the disturbance of ocular circulation but is safe to use and, as a result, found that a chymase inhibitor ameliorates a disturbance of ocular circulation and produces a prophylactic or therapeutic effect on diseases caused by such a disturbance of ocular circulation. The inventors have accordingly developed the present invention. The present invention, therefore, is directed to:
(1) A prophylactic or therapeutic medicament for the disease caused by a disturbance of ocular circulation which comprises a chymase inhibitor as the active ingredient.
(2) The prophylactic or therapeutic medicament as defined in the above paragraph (1) wherein the chymase inhibitor is an inhibitor of human chymase.
(3) The prophylactic or therapeutic medicament as defined in the above paragraph (1) which is in an ophthalmic topical dosage form.
(4) The prophylactic or therapeutic medicament as defined in the above paragraph (1) or (2) wherein the disease caused by a disturbance of ocular circulation is a disease selected from the group consisting of chorioretinal diseases and glaucoma.

The ohymase inhibitor which can be used in the present invention includes any and all substances that inhibit the enzymatic activity of chymases belonging to a subfamily of serine protease, and the use of inhibitors of human chymases such as human heart chymase, human mast cell chymase or human skin chymase are particularly preferred.

As substances having chymase-inhibitory activity, chymostatin and the following substances are already known.

Thus, USP 5314815 discloses an adduct of serine hydrolase with a phosphate or phosphonate represented by the formula (I) 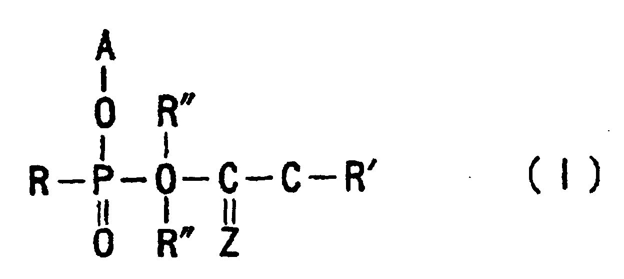 (wherein R represents an alkyl group or an alkoxy group, A represents a phenyl group or a naphthyl group, R' represents a phenyl group or an alkyl group, R" represents hydrogen or a methyl group, Z represents an electronegative group such as O, N, S or OH-NH₂). As specific compounds of the formula (I), there are mentioned 4-nitrophenyl phenaoyl methylphosphate, 4-nitrophenyl 4-nitrophenacyl methylphosphate, 4-nitrophenyl 4-methylphenacyl methylphosphate, 4-nitrophenyl 4-methoxyphenacyl methylphosphate and 4-nitrophenyl 4-chlorophenacyl methylphosphate.

USP 5306824 discloses a compound of the formula (II) 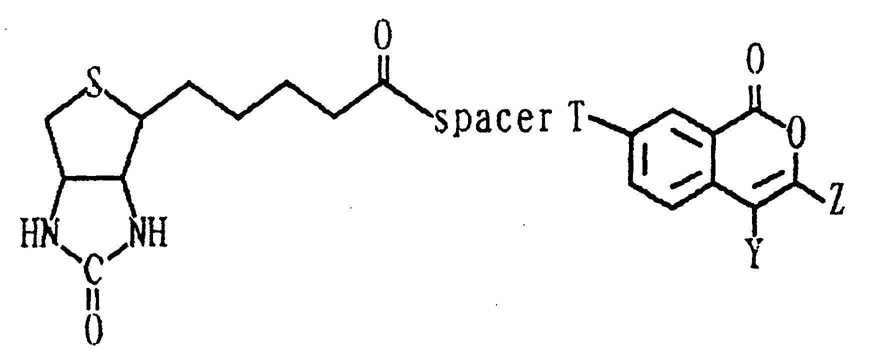 (wherein Z is selected from the class consisting of H, halogen, C₁₋₆ alkyl, C₁₋₆ fluorinated alkyl, C₁₋₆ alkyl substituted with R¹, C₁₋₆ fluorinated alkyl substituted with R¹, C₁₋₆ alkoxy, C₁₋₆ fluorinated alkoxy, C₁₋₆ alkoxy substituted with R¹, fluorinated alkoxy substituted with R¹, C₁₋₆ alkyl having a phenyl group, C₁₋₆ alkoxy having a phenyl group, C₁₋₆ alkyl having a phenyl group substituted with R², C₁₋₆ alkyl having a phenyl group substituted with two R² groups, C₁₋₆ alkoxy having a phenyl group substituted with R² group, and C₁₋₆ alkoxy having a phenyl group substituted with two R² groups, where R² represents halogen, COOH, OH, CN, NO₂, NH₂, C₁₋₆ alkyl, C₁₋₆ alkoxy, C₁₋₆ alkylamine, C₁₋₆ dialkylamine, C₁₋₆ alkyl-O-CO-, C₁₋₆ alkyl-O-CO-NH-, or C₁₋₆ alkyl-S-;
R¹ represents halogen, COOH, OH, CN, NO₂, NH₂, C₁₋₆ alkoxy, C₁₋₆ alkylamine, C₁₋₆ dialkylamine, C₁₋₆ alkyl-O-CO-, C₁₋₆ alkyl-O-CO-NH-, C₁₋₆ alkyl-S- or tosylamino; and the spacer is any organic structure which is 3-24 A long and including at least one linkage selected from the class consisting of -CH₂-CH₂-, -CO-NH-, -NH-CO-, -CH₂-CO-, -CH₂-NH-, -NH-CH₂- and -C₆H₄-; T represents -NH-, -O-, or -S-; and Y is selected from the class consisting of H, halogen, trifluoromethyl, methyl, OH and methoxy)

As specific examples, 7-biotinylamino-4-chloro-3-(2-phenylethoxy) isocoumarin and

### 7-(6-biotinylaminocaproyl)amino-4-chloro-3-(2-phenylethoxy)isocoumarin are mentioned.

WO 93/25574 discloses a compound of the formula (III) 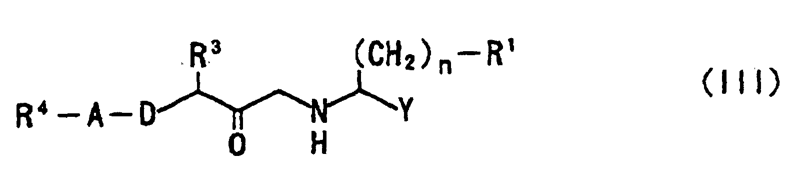 [wherein n is 1 or 2; R¹ is phenyl, naphthyl, (C₃₋₇)cycloalkyl, an unsaturated heterocycle group or a benzene ring-fused unsaturated heterocyclic group, where said unsaturated heterocyclic group is selected from among pyrrolyl, pyrrolinyl, furyl, dihydrofuryl, thienyl, dihydrothienyl, oxazolyl, oxazolidinyl, isoxazolyl, isoxazolinyl, imidazolyl, imidazolinyl, thiazolyl, thiazolidinyl, isothiazolyl, isothiazolinyl, pyrazolyl, pyrazolinyl, triazolyl, tetrazolyl, 1,3,5-oxadiazolyl, 1,2,4-oxadiazolyl, 1,3,5-thiadiazolyl, 1,2,4-thiadiazolyl, pyridyl, pyranyl, pyrazinyl, pyrimidinyl, pyridazinyl, 1,2,4-triazinyl, 1,2,3-triazinyl, 1,3,5-triazinyl, 1,2,5-thiadiazinyl, 1,2,5-oxathiazinyl and 1,2,6-oxathiazinyl; where said benzene ring-fused unsaturated heterocyclic group is selected from among benzoxazolyl, benzothiazolyl, benzimidazolyl, thianaphthenyl, isothianaphtenyl, benzofuranyl, isobenzofuranyl, chromenyl, isoindolyl, indolyl, indazolyl, isoquinolyl, quinolyl, phthalazinyl, quinoxalinyl, quinazolinyl, cinnolinyl and benzoxazinyl; where said phenyl, naphthyl, unsaturated heterocyclic group and benzene-fused unsaturated heterocyclic group may each optionally have 1∼3 substituents, which substituents may be independently selected from among bromo, chloro, fluoro, (C₁₋₅)alkyl, (C₁₋₅)alkoxy, (C₁₋₅)alkylthio, (C₁₋₅)alkylamino, (C₁₋₅)alkylsulfonyl, (C₁₋₅)dialkylamino, hydroxy, amino, nitro, cyano, trifluoromethyl, -COO(C₁₋₅)alkyl, -CONH₂, -CONH(C₁₋₅)alkyl, -CON=di(C₁₋₅)alkyl and formyl; where said (C₃₋₇)cycloalkyl may optionally have 1∼3 substituents, which substituents may be independently selected from among bromo, chloro, fluoro, (C₁₋₅)alkyl, (C₁₋₅)alkylthio, (C₁₋₅)alkoxy, hydroxy, trifluoromethyl and oxo(O=);
R³ represents (C₁₋₅)alkyl, (C₃₋₆)cycloalkyl, (C₃₋₆)cycloalkyl(C₁₋₅)alkyl, (C₁₋₅)alkoxy(C₁₋₂)alkyl, (C₁₋₅)alkylthio(C₁₋₂)alkyl, phenyl, unsaturated heterocyclyl, phenyl(C₁₋₂)alkyl, or unsaturated heterocyclyl(C₁₋₂)alkyl; where said unsaturated heterocyclyl is as defined above for R¹; where said unsaturated heterocyclyl(C₁₋₂)alkyl is partly comprised of the unsaturated heterocycle as defined above for R¹, an arbitrary one of the ring carbon atoms of the constituent unsaturated heterocycle being substituted with (C₁₋₂)alkyl; where said (C₁₋₅)alkyl, (C₃₋₆)cycloalkyl(C₁₋₅)alkyl and (C₃₋₆)cycloalkyl may each optionally be substituted with one or more fluorine atoms; where the rings of said phenyl, unsaturated heterocyclyl, phenyl(C₁₋₂)alkyl and unsaturated heterocyclyl(C₁₋₂)alkyl groups may each optionally have 1∼3 substituents, which substituents may independently be selected from among the functionalities mentioned as substituents on phenyl in the definition of R¹;
R⁴ is selected from the functionalities listed in the following categories (a)∼(d):
   a) piperazino, piperidino, pyrrolidino, 3-azabicyclo[3.1.0]-3-hexyl and azetidino, where the carbon atom or atoms of said piperazino may be optionally have 1 or 2 substituents independently selected from among (C₁₋₅)alkyl, (C₁₋₅)alkoxy(C₁₋₃)alkyl, hydroxy(C₁₋₃)alkyl, (C₁₋₅)alkylthio(C₁₋₃)alkyl, amino(C₁₋₃)alkyl, (C₁₋₅)alkylamino(C₁₋₃)alkyl and (C₁₋₅)dialkylamino(C₁₋₃)alkyl; where the 4-nitrogen atom of said piperazino may optionally be substituted with (C₁₋₅)alkyl, (C₁₋₅)alkoxy(C₂₋₄)alkyl, hydroxy(C₂₋₄)alkyl, amino(C₂₋₄)alkyl, (C₁₋₅)alkylamino(C₂₋₄)alkyl, (C₁₋₅)dialkylamino(C₂₋₄)alkyl and 2,2,2-trifluoroethyl; where any of the carbon atoms of said piperidino, pyrrolidino, 3-azabicyclo[3.1.0]-3-hexyl and azetidino may optionally have 1 or 2 substituents, which substituents may be independently selected from among chloro, bromo, fluoro, hydroxy, (C₁₋₅)alkyl, amino(C₁₋₃)alkyl, (C₁₋₅)alkylamino(C₁₋₃)alkyl, (C₁₋₅)dialkylamino(C₁₋₃)alkyl, (C₁₋₅)alkoxy(C₁₋₃)alkyl, (C₁₋₅)alkoxy, (C₁₋₅)alkoxy(C₁₋₃)alkoxy, amino, (C₁₋₅)alkylamino, (C₁₋₅)dialkylamino, (C₁₋₅)alkylthio, oxo(O=), unsaturated heterocyclyl, azetidino, pyrrolidino, piperidino, morpholino, 4-oxopiperidino, 4-hydroxypiperidino and piperazino, where the nitrogen in the 4 position of said piperazino may optionally be substituted with (C₁₋₅)alkyl, (C₁₋₅)alkoxy(C₂₋₄)alkyl, hydroxy(C₂₋₄)alkyl, amino(C₂₋₄)alkyl, (C₁₋₅)alkylamino(C₂₋₄)alkyl, (C₁₋₅)dialkylamino (C₂₋₄) alkyl, (C₁₋₅)dialkylamino(C₂₋₄)alkyl or 2,2,2-trifluoroethyl; where said unsaturated heterocyclyl is as defined above for R¹; where said unsaturated heterocyclyl may optionally have 1∼3 substituents selected independently from the funotionalities mentioned for the substituents on said unsaturated heterocyclic group in the definition of R¹;
   b) 4-morpholino, 4-thiomorpholino, 1-oxothiomorpholino or 1,1-dioxothiomorpholino; where the carbon atom or atoms of said 4-morpholino, 4-thiomorpholino, 1-oxothiomorpholino or 1,1-dioxothiomorpholino may optionally have 1 or 2 substituents, which substituents may be independently selected from among (C₁₋₅)alkyl, (C₁₋₅)alkoxy(C₁₋₃)alkyl, hydroxy(C₁₋₃)alkyl, (C₁₋₅)alkylthio(C₁₋₃)alkyl, amino(C₁₋₃)alkyl, (C₁₋₅)alkylamino(C₁₋₃)alkyl and (C₁₋₅)dialkylamino(C₁₋₃)alkyl,
   c) (C₁₋₇)alkyl or (C₃₋₇)cycloalkyl; where said (C₃₋₇)cycloalkyl may optionally have 1∼3 substituents which may be independently selected from among, halo, hydroxy, (C₁₋₅)alkoxy, (C₁₋₅)alkoxy(C₁₋₃)alkyl, hydroxy(C₁₋₃)alkyl, (C₁₋₅)alkylthio(C₁₋₃)alkyl, amino(C₁₋₃)alkyl, (C₁₋₅)alkylamino(C₁₋₃)alkyl, (C₁₋₅)dialkylamino(C₁₋₃)alkyl, (C₁₋₅)alkoxy(C₁₋₃)alkyloxy, amino, (C₁₋₅)alkylamino, (C₁₋₅)dialkylamino, (C₁₋₅)alkylthio, azetidino, pyrrolidino, piperidino, piperazino, 4-(C₁₋₅)alkylpiperadino, morpholino, thiomorpholino, oxothiomorpholino, dioxothiomorpholino, 4-oxopiperidino, 4-hydroxypiperidino and unsaturated heterocyclyl; where said unsaturated heterocyclyl is as defined above for R¹; where said unsaturated heterocyclyl may optionally have 1∼3 substituents selected independently from among the functionalities mentioned as substituents on said unsaturated heterocyclyl in the definition of R¹; where said (C₁₋₇)alkyl may optionally have 1∼3 substituents, which substituents may independently be selected from among halo, hydroxy, (C₁₋₅)alkoxy, (C₁₋₅)alkoxy(C₁₋₃)alkyloxy, amino, (C₁₋₅)alkylamino, (C₁₋₅)dialkylamino, (C₁₋₅)alkylthio, azetidino, pyrrolidino, piperidino, piperazino, 4-(N)-(C₁₋₅)alkylpiperazino, morpholino, thiomorpholino, oxothiomorpholino, dioxothiomorpholino, 4-oxopiperidino, 4-hydroxypiperidino and unsaturated heterocyclyl; where said unsaturated heterocyclyl is as defined above for R¹; where the unsaturated heterocyclyl may optionally have 1∼3 substituents selected independently from among the functionalities mentioned for the substituents on said unsaturated heterocyclic group in the definition of R¹;
   d) (R⁵E)-, wherein E represents oxygen, -NH or -N(C₁₋₅)alkyl, where R⁵ is (C₁₋₅)alkyl, 2,2,2-trifluoroethyl, R⁷(C₂₋₄)alkyl, R⁷CO(C₂₋₄)alkyl, 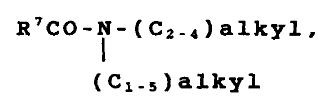 unsaturated amino(C₂₋₄)alkyl, amino(C₂₋₄)alkyl, (C₁₋₅)alkylamino(C₂₋₄)alkyl, (C₁₋₅)dialkylamino(C₂₋₄)alkyl, (C₁₋₅)dialkylamino(C₂₋₄)alkyl, (C₁₋₅)alkoxy(C₂₋₄)alkyl or hydroxy(C₂₋₄)alkyl; where the unsaturated heterocyclyl(C₂₋₄)alkyl is comprised of the unsaturated heterocycle defined for R¹, one of the ring atoms of which unsaturated heterocycle has been substituted with a (C₂₋₄)alkyl group; where said unsaturated heterocyclyl(C₂₋₄)alkyl may optionally have 1∼3 substituents independently selected from among the functionalities mentioned for the substituents on the unsaturated heterocyclic group in the definition of R¹;
R⁷ represents azetidino, pyrrolidino, piperidino, piperazino, 4-(N)-(C₁₋₅)alkylpiperazino, thiomorpholino, oxothiomorpholino, dioxothiomorpholino, or morpholino;
A represents carbonyl or sulfonyl;
D represents NH, N(C₁₋₅)alkyl, CH₂, oxygen, CH(OH) or CH-O-(C₁₋₅)alkyl;
X represents proline, 2-piperidinecarboxylic acid or 2-azetidinecarboxylic acid, where said proline, 2-piperidinecarboxylic acid and 2-azetidinecarboxylic acid may each optionally have 1 or 2 substituents, which substituents may be independently selected from among, bromo, chloro, fluoro, (C₁₋₅)alkyl, (C₁₋₃)alkoxy, oxo and hydroxy;
Y represents BF₂, B(OM)₂, -CO-Z or -C(OH)₂Z, where M represents hydrogen or (C₁₋₅)alkyl and the two substituents represented by M may, taken together with the boron atom and the two oxygen atoms to which they are attached, form a saturated heterocyclic group containing said boron atom, two oxygen atoms and 2 or 3 carbon atoms, where the carbon atom or atoms of said heterocyclic ring may optionally be substituted with 1 or two (C₁₋₅)alkyl groups;
Z represents CF₂R¹¹, 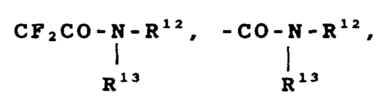 -CO-O-R¹²,
   or a heterocyclic groups selected from among 2-oxazolyl, 2-thiazolyl, 2-imidazolyl, 2-thienyl, 2-furyl, 2-pyrrolyl, 5-tetrazolyl, 2-benzothiazolyl, 2-benzoxazolyl, 2-benzimidazolyl, 2-benzofuryl, 2-benzothienyl and 2-indolyl; where said heterocyclic group may optionally have 1∼3 substituents, the substituent or substituents are independently selected from among (C₁₋₃)alkoxy, bromo, chloro, fluoro, (C₁₋₃)alkyl, hydroxy, amino, nitro, cyano, -COO(C₁₋₅)alkyl, -CONH₂, formyl, (C₁₋₅)alkylthio, (C₁₋₅)alkylamino, -CF₃, (C₁₋₄)alkyl-SO₂-, trifluoromethyl and (C₁₋₅)dialkylamino;
R¹¹ represents hydrogen, fluoro, (C₁₋₅)alkyl, (C₁₋₆)perfluoroalkyl, amino(C₁₋₅)alkyl, (C₁₋₅)alkylamino(C₁₋₅)alkyl, di(C₁₋₅)alkylamino(C₁₋₅)alkyl, (C₁₋₅)alkoxy(C₁₋₅)alkyl or hydroxy(C₁₋₅)alkyl;
R¹² and R¹³ each is independently selected from among hydrogen, (C₁₋₅)alkyl, (C₃₋₅)alkenyl, and R⁷(C₂₋₄)alkyl, where R⁷ is as defined above; provided, however, that (a) neither of the carbons alpha to the ring nitrogen of the substituent R⁴ should not be directly joined to the halogen, oxygen or nitrogen substituent, (b) when X is a substituted proline, 2-piperidineoarboxylic acid or 2-azetidinecarboxylic acid, none of fluoro, oxo, (C₁₋₃)alkoxy and hydroxy exist on the ring carbon adjacent to the nitrogen atom of said proline, 2-piperidinecarboxylic acid or 2-azetidinecarboxylic acid, and (c) the compound of the formula (III) cannot be a compound wherein n is 1, R¹ is phenyl, R³ is phenyl(C₁₋₂)alkyl, R⁴ is (R⁵E)-, where E is oxygen and R⁵ is (C₁₋₅)alkyl, A is carbonyl, D is NH, X is proline and Y is B(OM)₂]. As specific examples, the following compounds are mentioned.

N-[(1,1-dimethylethoxy)oarbonyl]-L-alanyl-N-[2,3-dioxo-3-methoxy-1-(phenylmethyl)propyl]-L-prolinamide;
N-[(1,1-dimethylethoxy)carbonyl]-L-valyl-N-[2, 3-dioxo-3-methoxy-1-(phenylmethyl)propyl]-L-prolinamide;
N-[4-[N-methylamino]piperidine-1-carbonyl]-L-valyl-N-[3,3,3-trifluoro-2-oxo-1(S)-(phenylmethyl) propyl]-L-prolinamide hydrochloride;
N-[4-[N-methylamino]piperidine-1-carbonyl]-L-valyl-N-[3,3,3-trifluoro-2-oxo-1(S)-(phenylmethyl)propyl]-L-prolinamide hydrochloride;
N-[4-[N-methylamino]piperidine-1-carbonyl]-L-valyl-N-[2,3-dioxo-3-((1-methyl)ethoxy)-1(S)-(phenylmethyl)propyl]-L-prolinamide hydrochloride;
N-[4-[N-methylamino]piperidine-1-carbonyl-L-valyl-N-[2,3-dioxo-3-((1-methyl)ethoxy)-1-(S)-(phenylmethyl)propyl]-L-prolinamide hydrochloride;
N-(4-oxopiperidine-1-carbonyl)-L-phenylalanyl-N-[2,3-dioxo-3-((1-methyl)ethoy)-1-(S)-(phenylmethyl)propyl]-L-prolinamide; and
N-[4-[N-methylamino]piperidine-1-carbonyl]-L-phenylalanyl-N-[3,3,3-trifluoro-2-oxo-1(S)-(phenylmethyl)propyl]-L-prolinamide.

WO 96/04248 discloses an imidazoline derivative of the formula (IV) 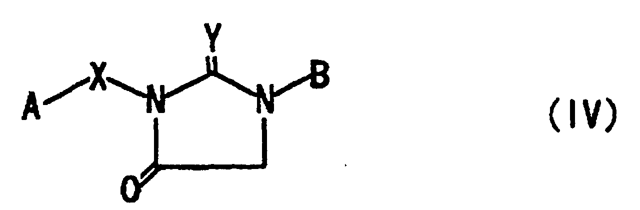 (A and B each represents an aromatic hydrocarbon group, which may be substituted by 1∼3 groups selected from among halogen, C₁₋₄ alkyl, C₁₋₄ alkoxy, C₁₋₄ alkylenedioxy, phenoxy, nitro, oyano, phenyl, C₂₋₅ alkanoylamino, carboxyl optionally esterified by C₁₋₄ alkyl or alkenyl, carboxyalkyl optionally esterified by C₁₋₄ alkyl or alkenyl, carboxyalkyloxy optionally esterified by C₁₋₄ alkyl or alkenyl, N-alkylpiperazinylcarbonyl, N-alkylpiperazinylcarbonylalkyl, N-alkylpiperazinylcarbonylalkyloxy and morpholinocarbonyl; X represents sulfonyl or carbonyl;
Y represents an oxygen atom or a sulfur atom), and as specific examples, the following species are mentioned:
3-(3-Methoxycarbonylbenzensulfonyl)-1-phenylimidazolidine-2,4-dione;
3-(4-Chlorobenzenesulfonyl)-1-phenyl-imidazolidine-2,4-dione;
3-(3,4-Dimethylbenzenesulfonyl)-1-(3,4-dimethylphenyl)-imidazolidine-2,4-dione;
3-(4-Allyloxycarbonylbenzenesulfonyl)-1-(3,4-dimethylphenyl)-imidazolidine-2,4-dione; and
1-(3,4-Dichlorophenyl)-3-(2-naphthylsulfonyl)imidazolidine-2,4-dione.

JP Kokai H9-31061 discloses a hydantoin derivative of the formula (V) 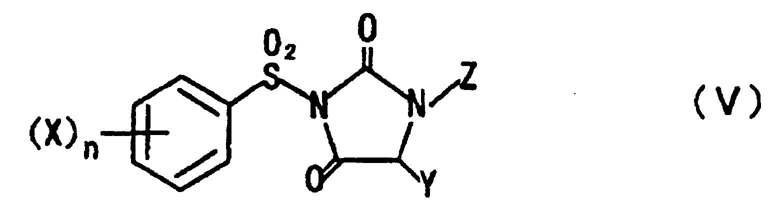 (wherein X represents a halogen atom, a lower alkyl group of 1~4 carbon atoms, a lower alkoxy group of 1∼4 carbon atoms or a carboxyl group optionally esterified by a lower alkyl group of 1∼4 carbon atoms or an allyl group, n represents an integer of 0∼5, Y represents a group of the formula (a) 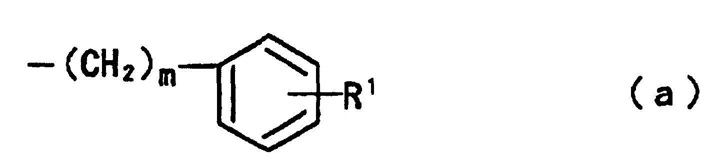 (wherein R¹ represents a hydrogen atom, a halogen atom, a hydroxyl group or a lower alkoxy group of 1~4 carbon atoms, m represents 0, 1 or 2), the formula (b)

―(CH₂)ₚ―OR² (b)

(wherein R² represents a hydrogen atom or a lower alkyl group of 1∼4 carbon atoms, p represents 1 or 2), or the formula (c)

―(CH₂)_{q}―COOR³ (c)

(wherein R³ represents a hydrogen atom or a lower alkyl group of 1∼4 carbon atoms, q represents 1 or 2, and Z represents a hydrogen atom, a lower alkyl group of 1∼4 carbon atoms, an aralkyl group of 7∼10 carbon atoms or a lower alkyloxycarbonyl group containing 2∼5 carbon atoms), and as specific examples, the following species, among others, are mentioned.
(5R)-5-Benzyl-3-(4-chlorobenzenesulfonyl)-imidazolidine-2,4-dione, and
(5R)-5-benzyl-3-(3,4-dimethylbenzenesulfonyl)-imidazolidine-2,4-dione.

WO 97/11941 discloses a quinazoline derivative of the formula (VI) 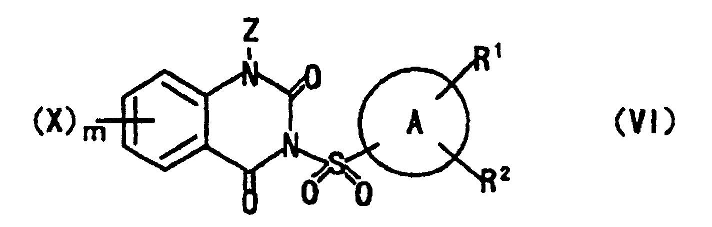 (wherein the ring A represents a benzene ring, a pyridine ring, a pyrrol ring or a pyrazole ring, m represents 0, 1 or 2, X represents a hydroxyl group, a nitro group, a halogen atom, a lower alkyl group of 1~4 carbon atoms which may optionally be substituted with halogon, a lower alkoxy group of 1~4 carbon atoms which may optionally be substituted with halogen, or an aralkyloxy group containing 7~12 carbon atoms, or X taken together with the benzene ring substituted therewith represents a naphthalene ring or a quinoline ring, R¹ and R² may be the same or different and each represents hydrogen, halogen, a lower alkyl group of 1∼4 carbon atoms which may optionally be substituted with halogen, a nitro group, a cyano group, a pyrazolyl group, a tetrazolyl group, a carboxyl group which may optionally be esterified with a lower alkyl group of 1∼4 carbon atoms or an allyl group, or a lower alkoxy group of 1∼4 carbon atoms which may optionally be substituted with 1 or more substituent groups selected from the class consisting of halogen, a morpholino group, a phenylpiperazinyl group and a carboxyl group which may optionally be esterified with a lower alkyl group of 1∼4 carbon atoms or an allyl group, or when the ring A is a benzene ring, R¹ and R², jointly and taken together with the benzene ring substituted therewith, constitute a naphthalene ring or a quinoline ring, Z represents hydrogen, a lower alkyl group of 1∼4 carbon atoms which may optionally be substituted with halogen, an alkenyl group of 2~5 carbon atoms, an aralkyl group which may optionally be substituted, an aromatic heterocycle-alkyl group which may optionally be substituted, a carboxymethyl group which may optionally be esterified with a lower alkyl group of 1∼4 carbon atoms or an allyl group, a carbonylmethyl group amidated with a primary, or secondary cyclic amine, an arylcarbonylmethyl group which may optionally be substituted, or an aralkyloxymethyl group which may optionally be substituted). As specific examples, the following compounds, among others, are mentioned.
[7-Chloro-3-(4-chlorobenzenesulfonyl)-2,4(1H, 3H)-quinazolinedion-1-yl]acetanilide,
[7-chloro-3-(4-chlorobenzenesulfonyl)-2,4(1H, 3H)-quinazolinedion-1-yl]acetic acid 4-hydroxyanilide,
[7-chloro-3-(4-chlorobenzenesulfonyl)-2,4(1H, 3H)-quinazolinedion-1-yl]acetic acid 3-pyridinamide.
[7-chloro-3-(4-chlorobenzenesulfonyl)-2,4(1H, 3H)-quinazolinedion-1-yl]acetic acid 4-pyridinamide,
[7-chloro-3-(4-chlorobenzenesulfonyl)-2,4(1H, 3H)-quinazolinedion-1-yl]acetic acid 2-pyridinamide,
3-{[7-chloro-3-(4-chlorobenzenesulfonyl)-2,4(1H,3H)-quinazolinedion-1-yl]acetylamino}-N-ethylpyridinium iodide,
[7-ohloro-3-(4-ohlorobenzenesulfonyl)-2,4(1H, 3H)-quinazolinedion-1-yl]acetic acid 5-indolamide,
3-{[7-chloro-3-(4-chlorobenzenesulfonyl)-2,4 (1H,3H)-quinazolinedion-1-yl]acetylamino)-(N-t-butoxycarbonylmethylpyridinium) bromide,
3-{[7-chloro-3-(4-chlorobenzenesulfonyl)-2,4(1H,3H)-quinazolinedion-1-yl]acetylamino}-N-carboxymethylpyridinium bromide,
3-[7-chloro-3-(4-chlorobenzenesulfonyl)-2,4(1H,3H)-quinazolinedion-1-yl]acetylaminophenoxy-phosphoric acid,
7-chloro-3-(4-chlorobenzenesulfonyl)-1-(3-pyridylmethyl)-2,4(1H,3H)-quinazolinedione, and
[7-chloro-3-(4-chlorobenzenesulfonyl)-2,4-(1H, 3H)-quinazolinedion-1-yl]acetic acid 4-chloroanilide.

EP 0713876 discloses a compound of the formula (VIIa) or (VIIb) 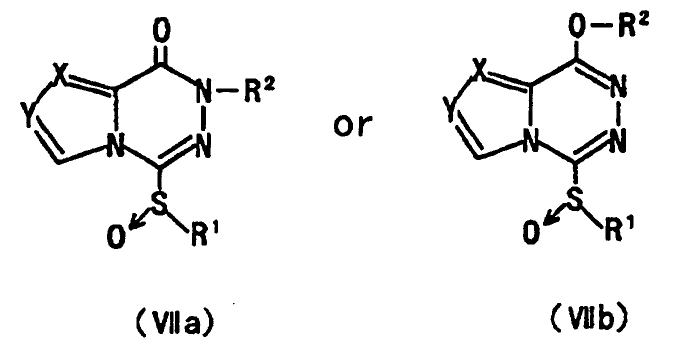 [In the formula (VIIa), assuming the case in which X represents C-CH₃ and Y represents N, R¹ represents a lower alkyl group or a benzyl group substituted with one halogen atom, R² represents a lower alkyl group, a benzyl group substituted with one halogen atom, or a lower alkoxycarbonylmethyl group. In the formula (VIIb), assuming the oases in which X represents N and Y represents CH, X represents CH and Y represents CH, and X represents C-CH₃ and Y represents N, R¹ represents a lower alkyl group, a lower alkoxycarbonylmethyl group, a phenyl - lower alkyl group, or a benzyl group, the benzene ring of which is substituted with one member selected from among lower alkyl, halogen, cyano, phenyl and halo(lower)alkyl and R² represents hydrogen, a lower alkyl, a lower alkoxycarbonylmethyl, a phenyl(lower)alkyl, or a benzyl group, the benzene ring of which is substituted with one member selected from among lower alkyl, halogen, cyano, phenyl and halo(lower)alkyl]. As specific examples, the following compounds are mentioned.
5-(4-Chlorobenzylsulfinyl)-8-hydroxyimidazo-[1,2-d][1,2,4]triazine,
8-(4-chlorobenzyloxy)-5-(4-chlorobenzylsulfinyl)imidazo[1,2-d][1,2,4]triazine,
8-(4-methylbenzyloxy)-5-(4-methylbenzylsulfinyl)imidazo[1,2-d][1,2,4]triazine,
8-(3-chlorobenzyloxy)-5-(3-chlorobenzylsulfinyl)imidazo[1,2-d][1,2,4]triazine,
1-(4-chlorobenzyloxy)-4-(4-chlorobenzylsulfinyl)-8-methylimidazo[1,5-d][1,2,4]triazine,
2-(4-chlorobenzyl)-4-(4-chlorobenzylsulfinyl)-8-methylimidazo[1,5-d][1,2,4]triazin-1(2H)-one,
5-(4-chlorobenzylsulfinyl)-8-ethoxycarbonylmethyloxyimidazo[1,2-d][1,2,4]triazine,
4-(4-chlorobenzylsulfinyl)-1-(ethoxycarbonylmethyloxy)-8-methylimidazo[1,5-d][1,2,4]triazine, and
2-ethoxycarbonylmethyl-4-(4-chlorobenzylsulfinyl)-8-methylimidazo[1,5-d] [1,2,4]triazin-1(2H)-one.

JP Kokai S10-87567 discloses a compound of the formula (VIII), inclusive of a salt thereof. 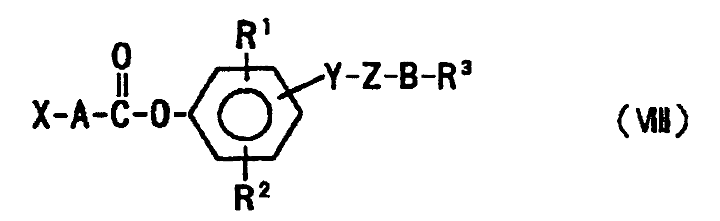 [wherein R¹ and R² may be the same or different and each represents hydrogen, a hydroxyl group, or a lower alkyl or lower alkoxy group which may be substituted, X represents a carbocyclic or heterocyclic group which may be substituted, A represents a bond or a lower alkylene group or imino group which may be substituted, Y represents a carbonyl group, a sulfonyl group, or a lower alkylene group or imino group which may be substituted, Z represents a phenylene group, bivalent heterocyclic group or imino group, which may be substituted, B represents a bond, a lower alkylene group or a phenylene group, R³ represents an acyl group or a carboxyl group which may be esterified or amidated]. As specific examples, the following compounds are mentioned.
1-[4-(3-Indolylacetoxy)benzoyl]-4-piperidine-carboxylic acid,
(S)-2-[4-(3-indolylacetoxy)benzoyl]-1,2,3,4-tetrahydroisoquinoline-3-carboxylic acid,
(S)-1-[4-(3-indolylacetoxy)benzoyl]-pyrrolidine-2-carboxylic acid, and
(S)-1-[4-(3-(2-methyl)indolylacetoxy)benzoyl]-pyrrolidine-2-carboxylic acid.

WO 96/33974 discloses a heterocyclic amide compound of the following formula (IX) or a pharmaceutically acceptable salt thereof. 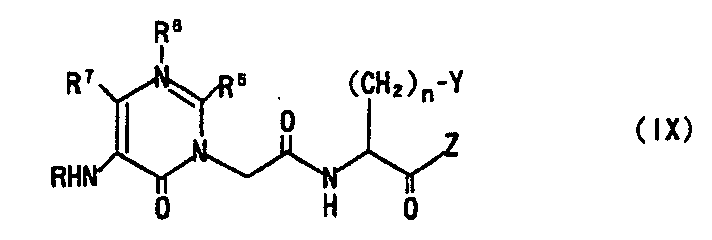 [wherein R represents hydrogen, -CHO, -CONH₂, -COR¹, -COOR¹, -CONHOR¹, -CONHR¹, -CONR¹R^{1'}, -CONHSO₂R¹, -COSR¹, -COCOR², -COCOOR², -CONHCOOR², -COCOMR³R⁴, -CSXR¹, -SO₂WR¹, -SO₂NR¹R¹' or -SO₂E (wherein R¹ and R¹' may be the same or different and each independently represents alkyl, cycloalkyl, cycloalkylalkyl, aryl, arylalkyl, heteroaryl, heteroarylalkyl, heterocyclyl or heterocyclylalkyl, R², R³ and R⁴ may be the same or different and each independently represents hydrogen, alkyl or arylalkyl, or the -NR³R⁴ group may as a unit represent a heterocyclyl group, X represents a direct bond, -NH-, -O- or -S-, W represents a direct bond, -NH-, -NHCO-, -NHCOO- or -NHCONH-, E represents hydroxy or amino),
R⁵, R⁶ and R⁷ may be the same or different and each independently represents hydrogen or alkyl, or one of R⁵, R⁶ and R⁷ represents aryl, arylalkyl, arylalkenyl, heteroaryl, heteroarylalkyl or heteroarylalkenyl, with the others each representing hydrogen, M represents carbon or nitrogen; provided, however, that when M is nitrogen, R⁶ does not exist, Y represents cycloalkyl, aryl or heteroaryl, z represents -CF₂R⁸, -CF₂CONR⁹R¹⁰, -CF₂COOR⁹, -COOR⁹ or -CONR⁹R¹⁰ (R⁸ represents hydrogen, halogen, alkyl, perfluoroalkyl, aminoalkyl, alkylaminoalkyl, dialkylaminoalkyl, alkoxyalkyl, hydroxyalkyl, aryl, arylalkyl, arylalkenyl, heteroaryl, heteroarylalkyl or heteroarylalkenyl,
R⁹ and R¹⁰ may be the same or different and each independently represents hydrogen, alkyl, alkenyl, cycloalkyl, cycloalkylalkyl, heterocyclylalkyl, aryl, arylalkyl, arylalkenyl, heteroaryl, heteroarylalkyl or heteroarylalkenyl, or -NR⁹ and R¹⁰ may jointly represent a heterocyclic group),
n represents 0 or 1. Among the above groups, the alkyl, cycloalkyl, cycloalkylalkyl, aryl, arylalkyl, arylalkenyl, heteroaryl, heteroarylalkyl, heteroarylalkenyl, heterocyclyl and heterocyclylalkyl may each be substituted]

As specific examples, the following compounds are mentioned.
2-[5-Amino-2-(3,5-diaminophenyl)-6-oxo-1,6-dihydro-1-pyrimidyl]-N-(1-benzyl-3,3,3-trifluoro-2-oxopropyl)acetamide and
2-(5-hydroxysuccinylamino-6-oxo-2-phenyl-1,6-dihydro-1-pyrimidyl)-N-(1-benzyl-3,3,3-trifluoro-2-oxopropyl)acetamide.

JP Kokai H9-124691 discloses a peptide compound of the following formula (X) or a pharmacologically acceptable salt thereof: 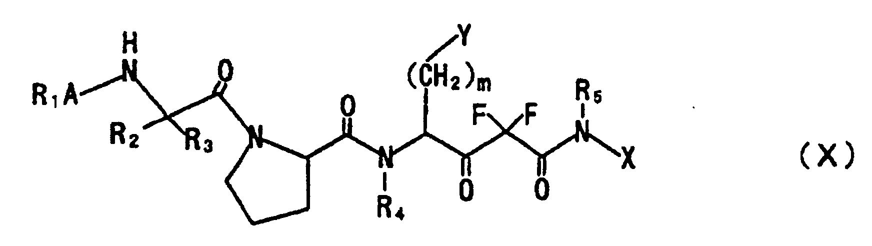 [wherein X represents a group of the formula 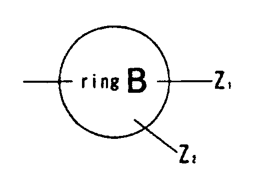 or 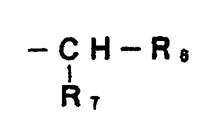 (wherein Z₁ and Z₂ may be the same or different and each represents hydrogen, hydroxy. a group of the formula -COR₈, alkyl which may optionally be substituted with the -COR₈ group, or a group of the formula -SO₂R₈,
R₆ and R₇ may be the same or different and each represents hydrogen, cycloalkyl, cycloalkylalkyl, aryl, arylalkyl, heteroaryl, heteroarylalkyl. a group of the formula -COR₈, or an alkyl, alkenyl, alkynyl, heterocyclyl or heterocyclylalkyl group substituted with -COR₈; provided, however, that R₆ and R₇ do not concurrently represent hydrogen and when either R₆ or R₇ or both represent heterocyolyl substituted with -COR₈, the hetero atom of said heterocyclyl being not attached to the carbon atom to which R₆ or R₇ is attached or R₆ and R₇ may jointly represent a methylene chain containing 4∼6 carbon atoms which may be substituted with -COR₈, the ring B represents an aromatic ring),
R₁ represents alkyl, cycloalkyl, cycloalkylalkyl, alkoxy, aryl, arylalkyl, heteroaryl, heterocyclyl or heterocyclylalkyl, which may be substituted with a group represented by -COR₈,
R₂ and R₃ may be the same or different and each represents hydrogen or an alkyl, cycloalkyl, cycloalkylalkyl. aryl or arylalkyl group which may be substituted with a group represented by -COR₈, or R₂ and R₃ may jointly represent a methylene chain of 4∼6 carbon atoms which may be substituted with a group represented by -COR₈,
R₄ represents hydrogen or alkyl,
R₅ represents hydrogen or alkyl, or when X is 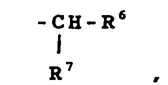 R₅ may be coupled to R₆ to form a methylene chain of 2∼4 carbon atoms,
R₈ represents hydroxy, alkoxy, amino, alkylamino, dialkylamino, aryloxy or arylalkoxy; Y represents cycloalkyl, aryl or heteroaryl,
m represents an integer of 1∼3 and A represents carbonyl or sulfonyl]

As specific examples, the following compounds are mentioned.
N-[4(S)-[N-[N-(tert-butyloxyoarbonyl)-L-valyl-L-prolyl]amino]-2,2-difluoro-3-oxo-5-phenylvaleryl] glycine,
3-[N-[4(S)-[N-[N-(tert-butyloxycarbonyl)-L-glutamyl-L-prolyl]amino]-2,2-difluoro-3-oxo-5-pheny lvalelyl]amino]benzoic acid, and
3-[N-[4(S)-[N-[N-(phenylsulfonyl)-L-glutamyl-L-prolyl]amino]-2,2-difluoro-3-oxo-5-phenylvaleryl] amino]benzoic acid.

JP Kokai H10-7661 discloses a heterocyclic amide compound of the following formula or its pharmacologically acceptable salt. 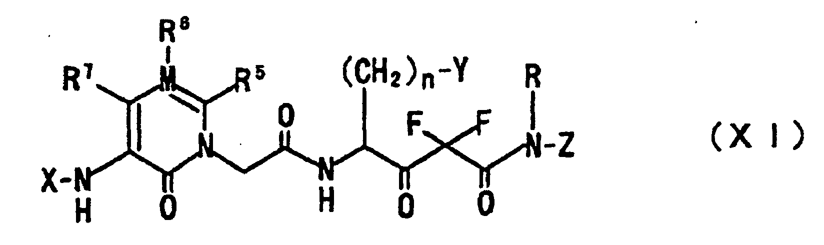 [wherein X represents -COOR¹, -(CH₂)ₘCOOR¹ or -CO(CH₂)ₘCOOR¹ (R¹ represents hydrogen, alkyl, cycloalkyl. cycloalkylalkyl, aryl, arylalkyl, heteroaryl, heteroarylalkyl, heterocyclyl or heterocyclylalkyl, m represents 1, 2 or 3), Z represents -(CH₂)ᵣCOOR² or a group of the formula (i) 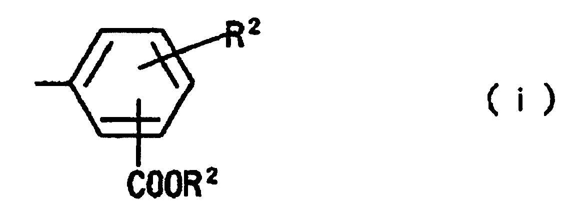 (wherein R² represents hydrogen, alkyl, cycloalkyl, cycloalkylalkyl, aryl, arylalkyl, heteroarylalkyl, heterocyclyl or heterocyclylalkyl, R³ represents hydrogen, alkyl, alkoxy or halogen, r represents 1, 2 or 3), R represents hydrogen or alkyl, R⁵, R⁶ and R⁷ may be the same or different and each independently represents hydrogen or alkyl, or one of R⁵, R⁶ and R⁷ represents aryl, arylalkyl, arylalkenyl, heteroaryl, heteroarylalkyl or heteroarylalkenyl, with the others each representing hydrogen, M represents carbon or nitrogen; provided, however, that when M is nitrogen, R⁶ does not exist, Y represents cycloalkyl, aryl or heteroaryl, n represents 0 or 1. Among the groups mentioned above, the aryl, arylalkyl, heteroaryl and heteroarylalkyl may each have a substituent or substituents]

As specific examples, the following compounds are mentioned.
2-[1,6-Dihydro-5-hydroxysuccinylamino-2-(3-methylphenyl)-6-oxo-1-pyrimidinyl]-N-[1-benzyl-3,3-difluoro-2-oxo-3-[N-(carboxymethyl)carbamoyl]-propyl]acetamide and
2-[1,6-dihydro-5-hydroxysuccinylamino-2-(3-methylphenyl)-6-oxo-1-pyrimidinyl]-N-[1-benzyl-3,3-difluoro-2-oxo-3-[N-(3-carboxyphenyl)carbamoyl]-propyl]acetamide.

JP Kokai H10-53579 discloses a compound of the following formula (XII) inclusive of its salt. 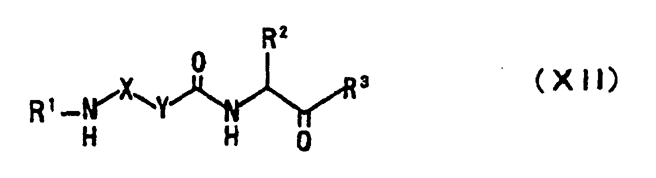 [wherein R¹ represents hydrogen or an amino-protecting group; R² represents an ar(lower)alkyl group; R³ represents a lower alkyl group which may optionally be substituted with one or more halogen atoms, a carboxyl group, or a protected carboxyl group; X represents a group of the following formula 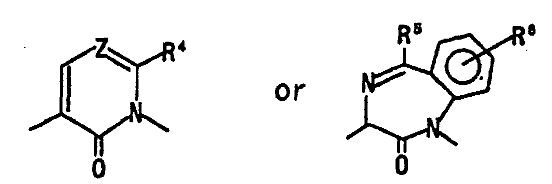 (wherein R⁴ and R⁵ each represents an aryl group which may optionally be substituted with 1 or more halogen atoms, lower alkoxy or phenyl; or a cyclo(lower)alkyl group; R⁶ represents hydrogen or a lower alkyl group; Z represents -N= or -CH=); Y represents a lower alkylene group]

As specific examples, the following compounds are mentioned.
2-[5-[(Benzyloxycarbonyl)amino]-2-(4-fluorophe nyl)-1,6-dihydro-6-oxo-1-pyrimidinyl]-N-[2-(4,4,4-t rifluoro-3-oxo-1-phenyl)butyl]acetamide,
2-[5-amino-2-(4-fluorophenyl)-1,6-dihydro-6-oxo-1-pyrimidinyl]-N-[2-(4,4,4-trifluoro-3-oxo-1-phenyl)butyl]acetamide,
2-[5-amino-2-phenyl-1,6-dihydro-6-oxo-1-pyrimidinyl]-N-[2-(4,4,4-trifluoro-3-oxo-1-phenyl) butyl]acetamide,
2-(5-amino-1,6-dihydro-6-oxo-2-phenyl-1-pyrimidinyl)-N-[2-(3-methoxycarbonyl-3-oxo-1-phenyl)butyl]acetamide, and
2-(3-amino-2,3-dihydro-2-oxo-5-phenyl-1H-1,4-benzodiazepin-1-yl)-N-[2-(4,4,4-trifluoro-3-oxo-1-phenyl)butyl]acetamide.

WO 98/09949 discloses an acetamide derivative of the formula (XIII). 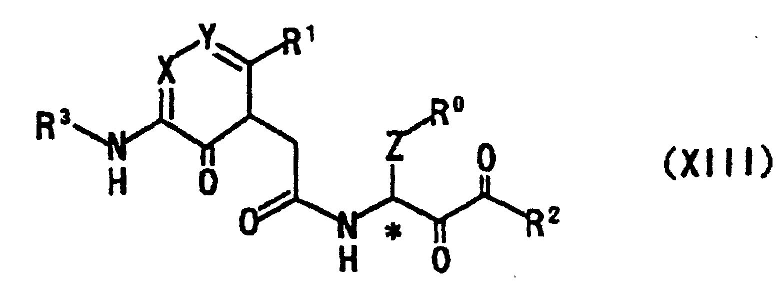 [wherein R⁰ represents a phenyl group which may have 1 or more nuclear substituents selected from the following groups collectively designated A (group A: the group A represents halogen, nitro, hydroxy, lower alkoxy, lower alkyl or halogen-substituted lower alkyl),
R¹ represents (i) aryl, (ii) heteroaryl, or (iii) straight-chain, branched-chain or cyclic C₁₋₆ alkyl, which may independently have one or more substituents selected from among the groups designated A above; or R¹ may have one or more substituents on any of said groups (i)-(iii), said substituents being selected from among the groups herein collectively designated B, namely ORₐ, COORₐ, CONR_{b}R_{c}, NR_{b}R_{c}, NR_{b}CHO, NR_{b}CORₐ, SO₂ORₐ, SO₂Rₐ, CONR_{b}SO₂Rₐ and P(O)(ORₐ)₂ (where Rₐ~R_{c} each independently represents hydrogen, lower alkyl or substituted lower alkyl; or Rₐ~R_{c} each independently represents aryl(C₁₋₇)alkyl, heteroaryl(C₁₋₇)alkyl, aryl or heteroaryl, among which the aryl or heteroaryl may nuclearly have one or more, usually 1∼3, substituents selected from among said groups designated A. The substituted lower alkyl has 1~3 atoms or groups selected from among halogen, nitro and hydroxy); or R¹ may have one or more substituents selected from among the cyclic groups herein collectively designated G on any of said groups (i)~(iii) (cyclic group G: the cyclic group G represents a 5- or 6-membered heterocyclic group containing oxygen or nitrogen within the range of 1∼3 atoms, which may optionally be substituted),
R² represents (C₁₋₈)alkyl, aryl(C₁₋₇)alkyl, heteroaryl(C₁₋₇)alkyl or aryl; or R₂ represents the above-defined group B or a (C₁₋₈)alkyl group substituted with the group B; or a (C₁₋₈)alkyl group substituted with the above-defined cyclic group G,
R³ represents hydrogen; or R³ represents an acyl group of the formula (i) D(CH₂)₀₋₃ · CO, (ii) D·CO·E·CO or (iii) D·SO₂·E·CO; or R³ represents a sulfonyl group of the formula D(CH₂)₀₋₃·SO₂ or D·CO·E·SO₂ (where D represents hydrogen, straight-chain, branched-chain or cyclic C₁₋₆ alkyl, aryl, halo-(lower)alkyl, halo(lower)alkoxy, amino, lower alkoxyamino, halo(lower)alkylamino, R_{b}R_{c}N, R_{b}R_{c}N·O, RₐO, Rₐ, RₐOCO, R_{b}R_{c}NCO, RₐSO₂NR_{b}, RₐS, or the cyclic group G defined above. The group E represents a bivalent bridging group of 1∼6 carbon atoms); or R³ represents a urea group of the formula R_{b}R_{c}NCO; or R³ represents a thiourea group of the formula R_{b}R_{c}N·CS; or R³ is Rₐ.

X and Y each independently represents a nitrogen atom or a carbon atom, which may be substituted with any of the groups represented by Rₐ~R_{c}.

Z represents a polymethylene group; the hydrogen atoms on the polymethylene group may independently be substituted with Rₐ or R_{b}]

As specific examples, the following compounds are mentioned.
2-(5-Amino-6-oxo-2-phenyl-1,6-dihydropyrimidin-1-yl)-N-{2,3-dioxo-1-phenylmethyl-6-(2-py ridyloxy)}hexylacetamide·2HCl,
2-{6-oxo-2-phenyl-5-(4-pyridylmethyloxycarbonylamino)-1,6-dihydropyrimidin-1-yl}-N-{2,3-dioxo-1-phenylmethyl-6-(2-pyridyloxy)}hexylacetamide,
2-(5-formylamino-6-oxo-2-phenyl-1,6-dihydropyrimidin-1-yl)-N-{2,3-dioxo-1-phenylmethyl-6-(2-pyridyloxy)}hexylacetamide, and
2-(5-benzylaminosulfonylamino-6-oxo-2-phenyl-1,6-dihydropyrimidin-1-yl)-N-(2,3-dioxo-1-phenylmethyl-6-(2-pyridyloxy)}hexylacetamide.

Furthermore, WO 98/18794 discloses a heterocyclio amide compound of the following formula, inclusive of its pharmacologically acceptable salt. 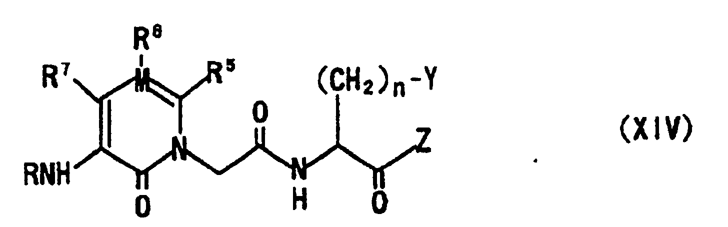 [wherein R represents hydrogen, alkyl, -COH, -CONH₂, -COR¹, -COOR¹, -CONHOR¹, -CONHR¹, -CONR¹R¹', -CONHSO₂R¹, -COSR¹,-COCOR², -COCOOR², -CONHCOOR², -COCONR³R⁴, -CSXR¹, -SO₂WR¹, -SO₂NR¹R¹' or -SO₂E (where R¹ and R¹' may be the same or different and each represents alkyl, cycloalkyl, cycloalkylalkyl, aryl, arylalkyl, heteroaryl, heteroarylalkyl, heterocyclyl or heterocyclylalkyl, R², R³ and R⁴ may be the same or different and each independently represents hydrogen, alkyl or arylalkyl, -NR³R⁴ may as a unit represent a heterocyclic group, X represents a single bond, -MH-. -O- or -S-, W represents a single bond, -NH-, -NHCO-, -NHCOO- or -NHCONH-, E represents hydroxy or amino), R⁵, R⁶ and R⁷ may be the same or different and each independently represents hydrogen or alkyl, or one of R⁵, R⁶ and R⁷ represents aryl, arylalkyl, arylalkenyl, heteroaryl, heteroarylalkyl or heteroarylalkenyl with the others each representing hydrogen, M represents a carbon atom or a nitrogen atom; provided, however, that when M is a nitrogen atom, R⁶ does not exist, Y represents cycloalkyl, aryl or heteroaryl, Z represents a group of the formula (i) 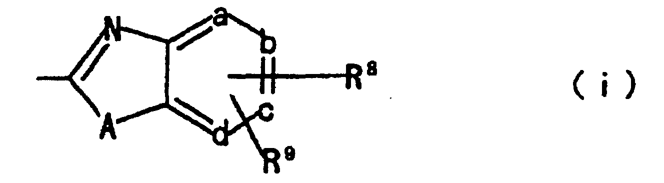 the formula (ii) 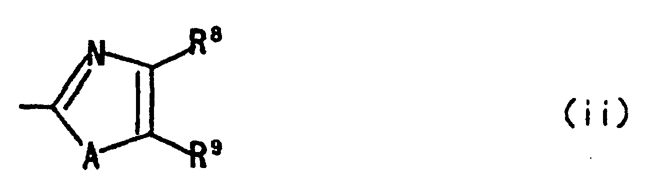 or the formula (iii) 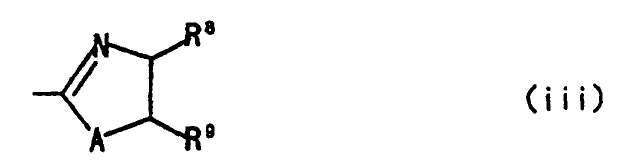 [wherein R⁸ and R⁹ may be the same or different and each independently represents hydrogen, alkyl, aryl, arylalkyl, heteroaryl, heteroarylalkyl, halogen, trifluoromethyl, cyano, nitro, -NR¹⁰R¹⁰', -NHSO₂R¹⁰, -OR¹⁰, -COOR¹⁰, -CONHSO₂R¹⁰ or -CONR¹⁰R¹⁰' (where R¹⁰ and R¹⁰' may be the same or different and each independently represents hydrogen, alkyl, cycloalkyl, cycloalkylalkyl, aryl, arylalkyl, heteroaryl, heteroarylalkyl or trifluoromethyl, or -NR¹⁰R¹⁰' may as a unit represent a heterocyclic group), A represents -O-, -S- or -NR¹²- (R¹² represents hydrogen, alkyl, cycloalkyl or cycloalkylalkyl), a, b, c and d each represents a carbon atom or any one of them represents a nitrogen atom with the others each representing a carbon atom], n represents 0 or 1.

Among the above groups, the alkyl, cycloalkyl, cycloalkylalkyl. aryl, arylalkyl, arylalkenyl, heteroaryl, heteroarylalkyl, heteroarylalkenyl, heterocyclyl and heterocyclylalkyl may optionally be substituted.

In the present invention, any of chymostatin and compounds of the above formulas (I)~(XIV) inclusive of their salts is used as the active ingredient. The salts include pharmaceutically or pharmacologically acceptable salts, for example alkali metal salts such as sodium salt, potassium salt, etc.; alkaline earth metal salts such as calcium salt, magnesium salt, etc.; salts with inorganic bases, such as aluminum salt, ammonium salt, etc.; salts with organic bases such as trimethylamine, pyridine, picoline, ethanolamine, diethanolamine, triethanolamine, dicyclohexylamine, N,N-dibenzylethylenediamine, etc.; salts with inorganic acids such as hydrochloric acid, hydrobromic acid, nitric acid, sulfuric acid, phosphoric acid, etc.; salts with organic acids such as formic acid, acetic acid, trifluoroacetic acid, fumaric acid, oxalic acid, tartaric acid, maleic acid, citric acid, succinic acid, malic acid, methanesulfonic acid, benzenesulfonic acid, p-toluenesulfonic acid, etc.; and salts with amino acids such as arginine, lysine, ornithine, aspartic acid, glutamic acid and so on.

The prophylactic or therapeutic medicament of the present invention can be formulated with a pharmaceutically acceptable carrier or excipient as needed and processed into per se known dosage forms by the known pharmaceutical technology involving a blending or dissolving procedure, for instance. Among such dosage forms, the form suitable for oral administration to human includes powders, granules, tablets, capsules, syrups and solutions, among others. The manufacture of solid dosage forms such as powders, granules and tablets can be carried out using those pharmaceutical carriers which are suitable for solid dosage forms, such as excipients (e.g. starch, glucose, fructose, sucrose, etc.), lubricants (e.g. magnesium stearate etc.), disintegrators (e.g. starch, crystalline cellulose, etc.), binders (e.g. starch, gum arabic, etc.) and so on. Such dosage forms may optionally be coated with a coating agent (e.g. gelatin, sucrose, etc.) or coated with an enteric film coating material (e.g. hydroxypropylmethylcellulose phthalate, a copolymer of methacrylic acid, shellac, etc.) so that the administered dosage form may dissolve selectively in the intestinal tract. When the intended dosage form is a syrup, a solution or the like, stabilizers (e.g. sodium edetate), suspending agents (gum arabic, carboxymethylcellulose, etc.), corrigents (simple syrup, glucose, etc.), flavoring agents, etc. can be selectively employed. The parenteral dosage form includes injections and suppositories. Preparation of injections can be made using solvents (e.g. distilled water for injection), stabilizers (sodium edetate, etc.), isotonizing agents (sodium chloride, glycerol, mannitol, etc.), pH control agents (hydrochloric acid, citric acid, sodium hydroxide, etc.) and suspending agents (methylcellulose etc.), among others.

The dosage form for topical administration to the eye includes various ophthalmic aqueous preparations such as ophthalmic aqueous suspensions, aqueous solutions, gels and emulsions. Moreover, non-aqueous ophthalmic preparations such as oily preparations, as well ophthalmic ointments and even long-acting or controlled-release topical ophthalmic preparations can be prepared.

For the manufacture of ophthalmic topical dosage forms, the known formulating additives such as solvents (physiological saline, purified water, etc.), chelating agents ( sodium edetate, citric acid, etc.), antioxidants (e.g. sulfurous acid and its salt), emulsifiers (e.g. polyvinylpyrrolidone), suspending agents and/or thickeners (e.g. celluloses such as hydroxypropylmethylcellulose, methylcellulose, etc., and high molecular compounds such as polyvinyl alcohol, polyvinylpyrrolidone, carboxyvinyl polymer, etc.), surfactants (e.g. nonionic surfactants such as Polysorbate 80, polyoxyethylene-hydrogenated castor oil, etc.), preservatives (quaternary ammonium salts such as benzalkonium chloride, benzethonium chloride, etc., parabens, and chlorobutanol), buffers (e.g. boric acid, borax, sodium acetate, citrate buffer, phosphate buffer, etc.), isotonizing agents (e.g. sodium chloride, glycerol, mannitol, sorbitol, etc.), pH control agents (e.g. hydrochloric acid and sodium hydroxide), etc. can be judiciously selected and used. Ophthalmic ointments can be prepared using white petrolatum, lanolin and other ointment bases.

The prophylactic or therapeutic medicament for a disturbance of ocular circulation according to the present invention is useful for the prophylaxis or therapy of chorioretinal diseases and glaucoma, in particular. The chorioretinal diseases include retinal vascular abnormalities such as retinal vascular occlusion, retinal periphlebitis, Eales' disease, ischemic eye syndrome, retinal arteriolar microaneurysm, etc., retinopathies caused by hypertension, renal disease or hemodyscrasia, diabetic retinopathy, retinal pigment epitheliopathy, retinal dystrophy, macular dystrophy, retinochoroidal atrophy, chorioretinopathy, macular degeneration, macular edema, detachment of retinal pigment epithelium, retinal detachment, degenerative retinoschisis, various tumors such as retinoblastoma, retinal pigment epithelioma, optic disc capillary hemangioma, etc., optic neuropathies such as ischemic optic neuropathy, and optic disc swelling such as choked disc and papilledema, among others. The glaucoma includes open-angle glaucoma, low-tension glaucoma and closed-angle glaucoma, among others.

The dosage of the prophylactic or therapeutic medicament of the present invention is dependent on the specific chymase inhibitor used as the active ingredient, the disease to be prevented or treated, clinical condition or status, the subject of treatment, and the method of administration, among other variables, and is not particularly restricted. The effective oral dosage of the compound of formula (IV) or a salt thereof may usually be 1∼1000 mg, preferably 10~500 mg, per dose and the effective parenteral dosage is usually 0.1~300 mg, preferably 1∼150 mg, per dose. For topical administration, an ophthalmic solution or suspension prepared at a concentration level of usually 0.001∼1.0 w/v %, preferably 0.01∼0.5 w/v %, is instilled in the eye, 20∼50 µl per dose about 5∼6 times daily.

The prophylactic or therapeutic medicament of the present invention may contain other active ingredients in addition to the chymase inhibitor. Such concomitant active ingredients include but are not limited to anticoagulants such as warfarin potassium, urokinase, aspirin, etc., vasoprotectant-hemostatics such as oarbazochrome sodium sulfonate etc., peripheral circulation improving agents such as kallidinogenase, pentoxifylline, sarpogrelate hydrochloride, tocopherol nicotinate, etc., adrenocorticosterides such as betamethasone, dexamethasone, predonisolone, etc., antiinflammatory enzymes such as serrapeptase, streptokinase, streptodornase, etc., antiglaucoma drugs such as β-blockers, mannitol, acetazolamide, anti-prostaglandin agent and so on.

### BRIEF DESCRIPTION OF THE DRAWING

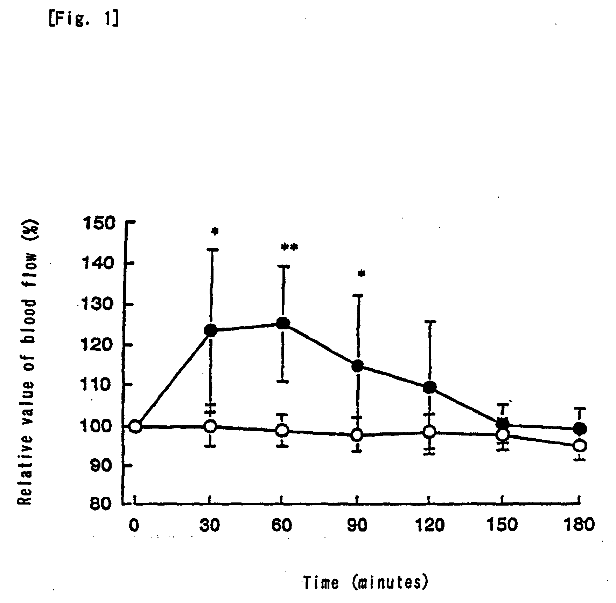

Fig. 1 shows changes in rabbit optic disc blood flow.

### BEST MODE FOR CARRYING OUT THE INVENTION

The following examples and test example are intended to describe the present invention in further detail and should by no means be construed as defining the scope of the invention.

### Example 1

**Tablets**

| | |
|---|---|
| 3-(4-Chlorobenzenesulfonyl)-1-phenylimidazolidine-2,4-dione | 200 mg |
| Lactose | 80 mg |
| Starch | 17 mg |
| Magnesium stearate | 3 mg |
| Crystalline cellulose | 10 mg |

Using the above formulation per tablet, tablets were prepared by the conventional procedure. Optionally, the tablets may be coated with a conventional enteric coating material (e.g. hydroxypropylmethylcellulose phthalate), a sugar-coating material or a film coating material (e.g. ethylcellulose).

### Example 2

### Capsules

| | |
|---|---|
| 3-(4-Chlorobenzenesulfonyl)-1-phenylimidazolidine-2,4-dione | 100 mg |
| Mannitol | 75 mg |
| Starch | 17 mg |
| Calcium stearate | 3 mg |

According to the above formulation per capsule, the ingredients were uniformly blended, granulated in the conventional manner, and filled into hard capsule shells. The granules for filling may be optionally coated with a conventional enteric coating material (e.g. hydroxypropylmethylcellulose phthalate), a sugar-coating material or a film coating material (e.g. ethylcellulose).

### Example 3

### Parenteral suspension for injection

| | |
|---|---|
| 3-(4-Chlorobenzenesulfonyl)-1-phenylimidazolidine-2,4-dione | 5 mg |
| Carboxymethylcellulose sodium | 500 mg |
| Distilled water for injection | To make 100 ml |

The above ingredients were aseptically blended in the conventional manner to give an injectable suspension.

### Example 4

### Ophthamic preparation (eye-drops)

| | |
|---|---|
| 3-(4-Chlorobenzenesulfonyl)-1-phenylimidazolidine-2,4-dione | 1.0 g |
| Sodium dihydrogenphosphate | |
| dihydrate | 0.1 g |
| Polysorbate 80 | 0.1 g |
| Sodium chloride | 0.9 g |
| Sodium hydroxide | q.s. |
| Sterilized pure water | To make 100 ml |

The above ingredients were blended in the conventional manner to give an ophthalmic suspension for instillation into the eye.

### Test Example 1

### Action on the rabbit optic disc blood flow Method

Six male pigmented rabbits (body weights: ca 2 kg) were used. Thus, 0.5% phenylephrine-0.5 tropicamide (Mydrin P, Santen Pharmaceutical Co.) was instilled into the rabbit eyes for mydriasis and the blood flow in the optic disc (optic nerve head) was measured, at the site selected avoiding the superficial vasoulature, using a laser speckle peripheral circulation analyzer. After measurement of the baseline blood flow, the ophthalmic preparation of Example 4 was instilled in one eye and the vehicle in the fellow eye, 50 µ l each. Blood flow was measured serially at 30-minute intervals up to 3 hours after instillation.

### Results

The results are presented in Fig. 1. In Fig. 1, the ordinate represents the relative value of blood flow (square blur rate) as measured by the laser speckle technique with the baseline value being taken as 100% and the abscissa represents the time after instillation of the ophthalmic preparation or the vehicle.
Furthermore,
● represents the mean ± standard deviation for the eyes given the ophthalmic preparation of Example 4,
○ represents the mean ± standard deviation for the eyes given the vehicle,
* denotes a significant difference at p<0.05 from the vehicle control eye as tested by paired t-test,
** denotes a significant difference at p<0.01 from the vehicle control eye as tested by paired t-test.

In the eye treated with the ophthalmic preparation of Example 4, an increased blood flow was noted in the optic nerve head and, compared with the control eye, the effect was significant at 30, 60 and 90 minutes after treatment.

The above results indicate that the chymase inhibitor as the active ingredient of the invention ameliorates a disturbance of ocular circulation.

### INDUSTRIAL APPLICABILITY

The prophylactic or therapeutic medicament for a disturbance of ocular circulation according to the present invention finds application in the prevention or treatment of retinal vascular abnormalities such as retinal vascular occlusion, retinal periphlebitis, Bales' disease, ischemic eye syndrome and retinal arteriolar microaneurysm, retinopathies associated with hypertension, renal disease or hemodyscrasia, diabetic retinopathy, retinal pigment epitheliopathy, retinal dystrophy, macular dystrophy, retinochoroidal atrophy, chorioretinopathy, macular degeneration, macular edema, detachment of retinal pigment epithelium, retinal detachment, degenerative retinoschisis, various tumors such as retinoblastoma, retinal pigment epithelioma, optic disc capillary hemangioma, etc., optic neuropathies such as ischemic optic neuropathy, optic disc swelling such as choked disc, papilledema, etc., glaucoma such as open-angle glaucoma, low-tension glaucoma and closed-angle glaucoma, and ocular hypertension associated with visual field loss.

## Claims

1. A prophylactic or therapeutic medicament for the disease caused by a disturbance of ocular circulation which comprises a ohymase inhibitor as the active ingredient.

2. A prophylactic or therapeutic medicament as set forth in Claim 1 wherein the chymase inhibitor is a human chymase inhibitor.

3. A prophylactic or therapeutic medicament as set forth in Claim 1 which is in an ophthalmic topical dosage form.

4. A prophylactic or therapeutic medicament as set forth in Claim 1 or 2 wherein the disease caused by a disturbance of ocular circulation is a disease selected from the group consisting of chorioretinal diseases and glaucoma.
